# EUROPEAN PATENT SPECIFICATION

(11) **EP 2 975 233 B1**
(45) Date of publication and mention of the grant of the patent: **06.06.2018**
(21) Application number: 14177713.6
(22) Date of filing: 18.07.2014
(51) Int. Cl.: F01N 3/20, C01B 3/04, C05G 3/00, H01M 8/0606, C01B 3/06, B01D 53/94, C01C 1/08, C05C 9/00, C12M 1/40, C12M 1/00

(54) **Ammonia precursor generating system for use in a vehicle**
Ammoniakvorläufererzeugungssystem zur Verwendung in einem Fahrzeug
Système de génération de précurseur d'ammoniac destiné à être utilisé dans un véhicule

(43) Date of publication of application: 20.01.2016
(73) Proprietor: Plastic Omnium Advanced Innovation and Research, 1120 Brussels (BE)
(72) Inventor: Dougnier, François, 3191 Hever (BE); Van Schaftingen, Jules-Joseph, 1300 Wavre (BE); Monge-Bonini, Beatriz, 1040 Bruxelles (BE)
(74) Representative: LLR

(56) References cited:
- WO-A1-2013/062410
- WO-A2-99/56858
- DE-A1- 4 425 420
- US-A1- 2004 040 288
- US-A1- 2004 126 294
- US-A1- 2008 286 165
- US-A1- 2009 087 891
- US-A1- 2011 138 780
- US-A1- 2012 189 923
- US-B1- 6 338 746

## Description

### Field of Invention

The invention relates to a tank comprising an ammonia precursor generating system which is capable of producing an ammonia precursor solution, and in particular an ammonia precursor boosting system for increasing the ammonia precursor concentration in an ammonia precursor liquid, for mounting on-board a vehicle.

### Background

There exist prior art systems for supplying ammonia or ammonia precursor to an exhaust line of a vehicle in order to reduce the NOx emissions. A SCR (Selective Catalytic Reduction) process is used for converting nitrogen oxides of an exhaust gas coming from a vehicle engine into diatomic nitrogen and water. The SCR process enables the reduction of nitrogen oxides by injection of a reducing agent, generally ammonia, into the exhaust line. This ammonia may be obtained by using different techniques.

One known technique is based on the use of an ammonia precursor, for example an aqueous urea solution. Generally, such urea solution is stored in a container mounted on the vehicle. The urea solution is injected into the exhaust line, and the gaseous ammonia is derived from the pyrolytic (thermal) decomposition of the injected urea solution. A problem with the known technique is that the urea concentration in the solution is relatively low, and that it cannot be increased without causing the freezing temperature of the urea solution to increase significantly.

Known SCR systems are injecting ammonia precursor such as Adblue® into the vehicle exhaust pipe. Adblue® is an Aqueous Urea Solution made with 32.5% by weight high-purity urea and 67.5% deionized water. The concentration of urea is limited to that level because it corresponds to an eutectic solution with a freezing point of -11°C. The AdBlue® remains liquid above this temperature but heating systems are required whenever temperatures are lower. Higher urea concentrations that would allow more compact storage and weight savings are not used today on vehicles, because freezing would start at even higher temperatures. AdBlue 40, which contains 40% by weight of urea starts freezing around 0°C already and is used in marine applications.

The consumption of AdBlue® (eutectic solution) for a vehicle complying with Euro 6.2 regulations is expected to be around 0.15 l/100 km, corresponding to a capacity requirement of 45 litres if the driving range of the vehicle has to be 30000 km (maintenance interval), and the weight of the full system would be around 60 kg. Such large volume is very difficult to handle in a passenger car, and weight is penalizing the performances of the car, both in terms of pollution (CO2) and dynamics. A vehicle that would be powered by AdBlue® (used as a fuel, for instance by conversion of the urea to ammonia or to hydrogen that feed a fuel cell generating electrical power) would consume around 28 litre of AdBlue® (eutectic solution) per 100 km. A typical tank that can fit the environment of a passenger car (say 70 litres), would therefore allow a driving range of 250 km, what is reasonable compared to today's electrical vehicles based on batteries, but still very low compared to current vehicles based on conventional fuels. The increase of driving range would require much larger tanks, for instance 210 litres for 750 km, what is practically impossible in terms of car architecture and weight. One can think of increasing the concentration of urea above 32.5 % by weight. However the freezing point would become larger than -11°C; for instance, increasing the urea concentration from 32.5 to 44% will increase the freezing point from -11°C to 7°C, a normal winter temperature in many countries where AdBlue® is commercialized. This increase in freezing temperature will prevent convenient handling of AdBlue®.

United States Patent Application US2004/0126294 discloses the generation of ammonia by hydrolysis of an aqueous urea solution obtained by supplying urea as dry solid into a dissolver for solubilizing with water and by adding an additional water amount for adjusting the desired concentration as well as the supply of this generated ammonia to an SCR or SNCR process for removal of nitrogen oxide from combustion gases.

### Summary

The object of embodiments of the invention is to provide an ammonia precursor generating system which is capable of producing an ammonia precursor solution having a higher ammonia precursor concentration compared to prior art solutions whilst maintaining an acceptable handling, and in particular an ammonia precursor boosting system for increasing the ammonia precursor concentration in an ammonia precursor liquid.

According to a first aspect of the invention there is provided a tank according to claim 1 comprising an ammonia precursor generating system comprising a storage compartment storing at least ammonia precursor granules; a dissolving compartment adapted for storing an ammonia precursor solution, and for dissolving ammonia precursor granules in the ammonia precursor solution; and a transfer means configured for transferring ammonia precursor granules from said storage compartment to said dissolving compartment.

In that way, by adding granules from the storage compartment, the concentration of ammonia precursor in the ammonia precursor liquid in the dissolving compartment can be increased when needed. The granules can be stored safely in the storage compartment without increasing the freezing point of the ammonia precursor solution, and it is only when the ammonia precursor is needed and when the temperature in the dissolving unit is sufficiently high that granules will be added.

In other words, embodiments of the invention have the advantage of allowing an increase of the ammonia precursor concentration, which reduces necessary volumes and weights of storage, whilst at the same time keeping low temperature freezing points. It is especially useful for automotive applications as a booster, for systems consuming urea or urea derivatives such as ammonia for SCR applications, or as fuel for fuel cells.

The transfer means may be e.g. a pump, a valve, a combination of both, gravity, gravity in combination with a valve, or a valve in combination with whatever system known by the person skilled in the art to transfer granules, optionally in a liquid. In a preferred embodiment the transfer means is a dosing device configured for dosing a number of ammonia precursor granules to be inserted in the dissolving compartment. In that way the adding of granules, and hence the increase of the concentration of the ammonia precursor in the solution, can be controlled in an accurate manner.

In a preferred embodiment the dissolving compartment comprises a trigger device arranged for triggering the dissolving of the ammonia precursor granules in the ammonia precursor liquid. This trigger device may be a thermal trigger, a chemical trigger, a mechanical trigger, and more generally any trigger device capable of causing the dissolution of the granules in the ammonia precursor solution. In a particular embodiment the trigger device is a heater arranged for heating the ammonia precursor solution in the dissolving compartment and configured for dissolving the ammonia precursor granules in the ammonia precursor liquid.

In a preferred embodiment the storage compartment stores ammonia precursor granules containing solid urea.

In an exemplary embodiment the storage compartment stores ammonia precursor granules having a coating, said coating being adapted to be dissolved, preferably thermally dissolved, in the ammonia precursor liquid. For instance, granules containing solid urea in a protective shell can be added to eutectic AdBlue®, and be dissolved in the dissolving compartment, optionally heated at higher temperatures, e.g. between 40 and 60 °C, preferably just before being consumed, i.e. before being sent to the exhaust pipe for SCR applications or before being converted, for instance to ammonia or aqua ammonia for use in SCR systems or fuel cells. The use of protected urea granules in addition to AdBlue® or water or intermediate concentration solutions, will increase the ammonia concentration in the fluid used by the SCR system or fuel cells, and consequently increase the drive range. The protected granules will release urea upon reaching targeted conditions.

In an exemplary embodiment, the coating of the ammonia precursor granules may be made of any one or more of the following materials: polyvinylidene chloride (PVDC), linear low density polyethylene (LLDPE), certain grades of ethylene vinyl alcohol (EVOH), certain grades of polyvinyl alcohol (PVOH), bi-axially oriented polypropylene (BOPP), cyclic olefin polymer (COC), polyethylene naphthalate (PEN), liquid-crystal polymers (LCPs, a class of aromatic polyester polymers), polypropylene (PP), and polyethylene terephthalate blends (PET/PE, PET/PVDC/PE, PET/PVOH/PE, PET/EVOH/PE). Suitable examples of coating materials can be found in the packaging industry. The material(s) may be chosen such that the coating is thermally dissolved in the ammonia precursor solution when the temperature is within a certain range, e.g. above 40 degrees Celsius. The coating may be a single layer coating or a multi-layer coating.

In a possible embodiment the storage compartment stores the ammonia precursor granules in a liquid, typically an ammonia precursor liquid and the coating is such that the coating is not dissolved in the liquid in the storage compartment. In that way, ammonia precursor solution and granules may be added together to the storage compartment, e.g. via the same filler pipe.

In an exemplary embodiment the system further comprises a filler pipe in connection with the storage compartment for filling the storage compartment with ammonia precursor granules, and optionally also with an ammonia precursor liquid. In the latter case the storage compartment may be arranged for allowing said ammonia precursor liquid to be transferred to the dissolving compartment.

In a further developed embodiment the dissolving compartment may be provided with a decomposition activator device configured to convert ammonia precursor solution to ammonia solution in the dissolving compartment. The decomposition activator device may comprise an enzyme storage unit configured to store an enzyme, and an enzyme transfer means configured for transferring enzyme to the dissolving compartment, said enzyme being adapted to convert ammonia precursor to ammonia. Note that the ammonia solution after the conversion of the ammonia precursor solution may comprise other compounds than ammonia (hydrated ammonia / ammonium hydroxide), water and carbon dioxide, such as impurities or other effluents. E.g. if the mixture is obtained by decomposition of an aqueous urea solution then it may also comprise a residue of an ammonia precursor (e.g. a portion of the ammonia precursor that has not been decomposed) and other compositions, such as ammonium hydrogen carbonate.

More generally the decomposition activator may be a bio-agent 3 (preferentially an enzyme, urease) to obtain an NH3/CO2 mixture which may be an aqueous solution which may be a liquid and/or a liquid with particles in suspension. The bio-agent may be thermally activated by a heater. Such an example of a urea decomposition unit is disclosed in patent applications EP 13182919.4 and EP 12199278.8 in the name of the Applicant, the contents of which are included herein by reference. In those applications the Applicant has proposed two new methods for generating ammonia on board a vehicle (passenger car, truck, etc.) based on a biological catalysis. Biological catalysis comprises all forms of catalysis in which the activating species (i.e. biological catalysts) is a biological entity or a combination of such. Included among these are enzymes, subcellular organelles, whole cells and multicellular organisms. More precisely, according to a first method, a protein component is used to catalyze the hydrolysis (i.e. decomposition) of an ammonia precursor solution (for example, urea) into a mixture comprising at least ammonia, carbon dioxide and water. Such first method is described in more detail in patent application EP 13182919.4. According to a second method proposed by the Applicant, a protein component is used to catalyse the hydrolysis (i.e. decomposition) of an ammonia precursor solution (for example, urea) into ammonia gas. For example, the generated ammonia gas can be directed (i.e. transmitted) to a solid absorbing matrix where it is stored thereon by sorption. Such second method is described in more detail in patent application EP 12199278.8.

According to another exemplary embodiment the ammonia precursor generating system comprises a separate decomposition compartment provided with a decomposition activator device configured to convert ammonia precursor solution to an ammonia solution in the decomposition compartment, and a transfer means configured for transferring ammonia precursor solution from the dissolving compartment to the decomposition compartment. The decomposition activator device may comprise an enzyme storage unit configured to store an enzyme, an enzyme transfer means configured for transferring enzyme to the decomposition compartment, said enzyme being adapted to convert ammonia precursor to ammonia, and a heater.

In an exemplary embodiment with a decomposition activator device, the system may further comprise a buffer compartment for storing the ammonia solution. The buffer compartment may be integrated in the same module as the dissolving compartment. Preferably the buffer compartment surrounds the dissolving compartment.

The ammonia solution with increased or "boosted" concentration in the buffer tank is ready to be sent a downstream tank, to an exhaust pipe or to any additional system storing or consuming ammonia. In a possible embodiment the system further comprises a conversion unit for converting ammonia into hydrogen. The ammonia-hydrogen conversion unit may subsequently communicate with a hydrogen fuel cell where the hydrogen is converted into a power source. The ammonia solution could also be used in a direct ammonia fuel cell. The storage compartment and the dissolving compartment are arranged in a common tank. The common tank is storing an ammonia precursor solution, such as for instance urea or a concentrated urea solution of at least 10% urea up to the eutectic 32.5 wt% urea in water.

In a preferred embodiment the storage compartment stores ammonia precursor granules having dimensions between 0.01 micron and 50 mm, more preferably between 100 micron and 5 mm, and e.g. between 500 microns and 5 mm.

### Brief description of the figures

The accompanying drawings are used to illustrate presently preferred non-limiting exemplary embodiments of devices of the present invention. The above and other advantages of the features and objects of the invention will become more apparent and the invention will be better understood from the following detailed description when read in conjunction with the accompanying drawings, in which:
Figure 1 is a diagram illustrating an exemplary embodiment of a vehicle system for generating hydrogen for a hydrogen fuel cell;
Figures 2, 3, 4, 5, and 6 are diagrams illustrating ammonia precursor generating and decomposition systems;
Figures 7A-C illustrate two variants of a module for use in an exemplary embodiment of an ammonia precursor generating and decomposition system; and
Figures 8 and 9 are diagrams illustrating further exemplary embodiments of an ammonia precursor generating and decomposition system.

### Description of embodiments

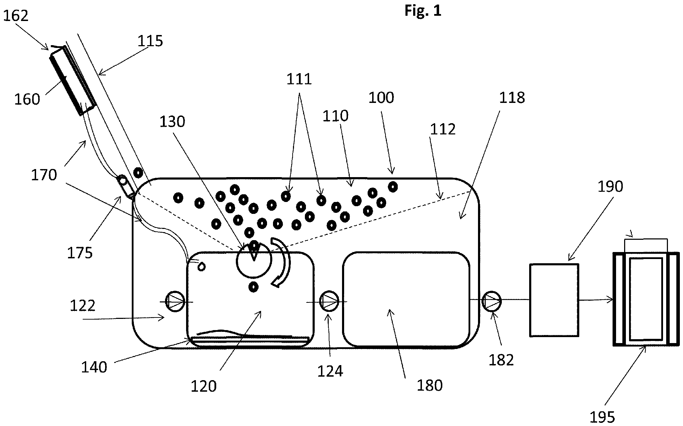

Figure 1 illustrates a first embodiment of an ammonia precursor booster system as used in a vehicle system for generating hydrogen for a hydrogen fuel cell. The ammonia precursor booster system comprises a storage compartment 110 in the form of an inner chamber of a tank 100, a dissolving compartment 120 in the form of a sub-tank located in the tank 100, and a transfer means 130. The storage compartment 110 is adapted for storing ammonia precursor granules 111. The dissolving compartment 120 is adapted for storing an ammonia precursor solution, and for dissolving ammonia precursor granules in the ammonia precursor solution. The transfer means 130 are configured for transferring ammonia precursor granules from the storage compartment 110 to the dissolving compartment 120. In the embodiment of figure 1 the transfer means is a dosing device 130 configured for dosing a number of ammonia precursor granules to be inserted in the dissolving compartment 120.

The dissolving compartment comprises a trigger device in the form of a heater 140 arranged for triggering the dissolving the ammonia precursor granules 111 in the ammonia precursor liquid in the dissolving compartment 120.

In the present embodiment the dissolving compartment 120 also functions as a decomposition unit for converting ammonia precursor into ammonia. There is provided a decomposition activator device comprising an enzyme storage unit 160 configured to store an enzyme, and an enzyme transfer means 170, 175 configured for transferring enzyme to the dissolving compartment 120, said enzyme being adapted to convert ammonia precursor to ammonia. The enzyme storage unit 160 has an enzyme access port 162 for filling the enzyme storage unit 160 with enzyme. The heater 140 functions also as a part of the decomposition activator device for activating the enzymatic conversion of ammonia precursor to ammonia in the dissolving compartment 120.

The system of figure 1 further comprises a filler pipe 115 in connection with the storage compartment for filling the storage compartment with ammonia precursor granules, and for filling said storage compartment with a ammonia precursor liquid, wherein the storage compartment 110 is arranged for allowing said ammonia precursor liquid to flow to the dissolving compartment 120, here by providing the storage compartment 110 above the dissolving compartment 120 and by providing the bottom of the storage compartment 110 with through holes allowing the liquid to pass through whilst blocking the granules.

Also the system of figure 1 comprises a buffer compartment 180 for storing converted ammonia, a conversion unit 190 for converting ammonia into hydrogen, and a hydrogen fuel cell 195.

The tank 100 may be filled with the commercially available liquid ammonia precursor, known as AdBlue® and matching the ISO 22241 standard specifications. Such a fluid contains 32.5 ± 0.7 weight % urea. The tank 100 is equipped with a chamber forming the storage compartment 110 containing small granules, e.g. ball-shaped granules containing solid urea, in order to increase the urea concentration in the solution to be converted into ammonia solution. The urea granules are preferably coated so as to avoid the release of the solid urea in the main chamber 118 of the tank that could result in a premature increase of the urea concentration and precipitates in or freezing of the content of the main chamber 118. Preferably, the size of the ammonia precursor granules is in a range from 0.01 micron to 50 mm, more preferably from 100 micron to 5 mm, and e.g. between 500 microns and 5 mm

When conversion from urea to ammonia is needed, the urea solution is transferred from the main chamber 118 to the decomposition unit 120 by a fluid transfer device 122. A fluid transfer device can be for example a pump, a valve, a combination of both, gravity, gravity in combination with a valve, or a valve in combination with whatever system known by the person skilled in the art to transfer liquid. The decomposition unit 120 is equipped with a heater 140 in order to thermally dissolve the coating of the ammonia precursor granules, dissolve the solid urea contained in the granules, and activate the enzymes converting the urea to ammonia. The ammonia precursor granules can be transferred to the decomposition unit 120 by a dosing device 130. The dosing device may be a device as described in US 20070128054A1 in the name of the Applicant, a dosing device as described in FR2911639B or FR2911641B, a powder dosing device, such as described in EP0296632A2, EP0859944B1, EP0973015A1, US6701944B2, US0318218A1 or US6510962B1, etc. When conversion is needed, the dosing device 130 is activated and a small quantity of enzyme is transferred from the enzyme storage unit 160 to the decomposition unit 120 by means of a connecting pipe 170 and a dosing pump 175. The enzyme storage unit 160 contains a bio-agent suitable to convert the urea solution into an ammonia solution. For example, as an enzyme, urease can be used to decompose urea, or any other suitable protein sequence. As the temperature remains relatively high in the decomposition unit 120, no freezing or precipitating of urea occurs. Also, once the urea is converted into ammonia, no freezing occurs either as the freezing temperature of the effluents from the decomposition is even lower.

When the conversion is complete, for instance, when 50% of the ammonia precursor, typically urea, is converted into an ammonia solution, or ideally when at least 80 % or more of the ammonia precursor is converted into aqua ammonia, the resulting effluents are transferred to the buffer tank 180 by a fluid transfer device 124. Thus, the system of the embodiment of figure 1 allows increasing the urea concentration in the urea solution to be converted to ammonia and, consequently, increasing the ammonia concentration in the final solution.

The ammonia solution with increased or "boosted" concentration in the buffer tank 180 is ready to be sent by a fluid transfer device 182 to a downstream tank, to the exhaust pipe or to any additional system to store or consume ammonia (not illustrated). In the example shown in figure 1, the ammonia buffer tank 180 communicates with an ammonia-hydrogen conversion unit 190 that subsequently communicates with a hydrogen fuel cell 195 where the hydrogen is converted into a power source. The ammonia solution could also be used in a direct ammonia fuel cell (not illustrated).

The filler pipe 115 is connected to an inner chamber forming the storage compartment 110. The inner chamber has holes 112, allowing the liquid ammonia precursor to flow inside the lower part of tank 100 (the main chamber 118) but being too small for letting the granules flow into the main chamber 118 of the tank. The inner chamber 110 is connected to the decomposition unit 120 through the dosing device 130, so that granules containing solid urea can be introduced at the top of the decomposition unit 120. The migration of the granules 111 to the inner chamber 110 can be promoted by the movement of the ammonia precursor liquid when the tank is refilled, but this is not required.

In an exemplary embodiment, the ammonia precursor granules 111 can be transferred to the decomposition unit 120 after the conversion to ammonia is partially completed, for instance, when at least 30% of the ammonia precursor is converted into ammonia solution. The presence of ammonia in the solution may help to chemically decompose the coating of the granules.

In variants of the embodiment of figure 1 other means for releasing the solid urea contained in the granules may be used, in addition or as an alternative to temperature and chemical attack. Possible other means comprise the use of a mechanical force, or a pressure, or a combination of any of those forms or any other means known in the art.

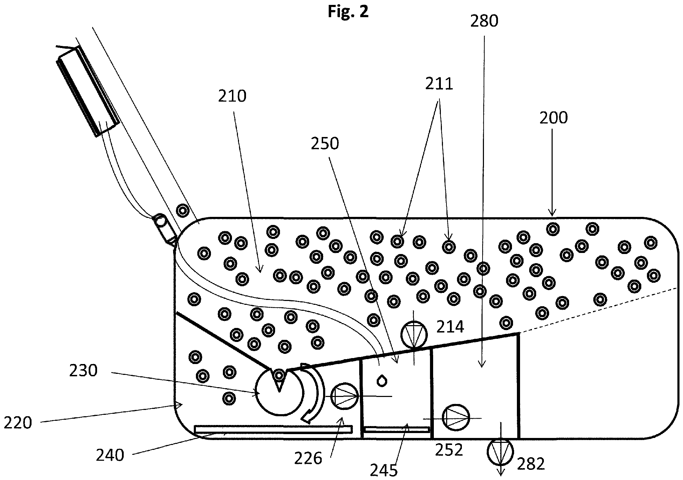

Figure 2 illustrates another ammonia precursor generating system in which ammonia precursor granules 211 are transferred from a storage compartment 210 of a tank 200 to a dissolving compartment in the form of a warm chamber 220 to be dissolved, before being decomposed in a decomposition unit 250. The warm chamber 220 is equipped with a heater 240 in order to thermally dissolve the coating material and release the urea content, and the decomposition unit 250 is provided with a further heater 245 for the conversion into ammonia. This physical separation between the dissolving compartment 220 and the decomposition unit 250 limits the temperature interference that can happen between the two different compartments, since the temperature for enzyme activation and coating dissolution may be different: for instance, the dissolution temperature of the coating may be 70°C, while the optimum temperature for the decomposition unit may be 50°C. The boosted ammonia solution may be cooled down by a cooling device, which can be natural heat dissipation occurring between the warm chamber 220 and the decomposition unit 250 at the level of a fluid transfer device 226, and then transferred to the decomposition unit 250 by the fluid transfer device 226. Dosing device 230 transfers both granules and ammonia precursor liquid to the warm chamber 220. Further, ammonia precursor liquid may be fed into the decomposition unit 250 via an optional fluid transfer device 214. Next the ammonia solution may be transferred to a buffer compartment 280 via a further fluid transfer device 252, and from there via a fluid transfer device 282 to e.g. the exhaust pipe of a vehicle. In a possible embodiment fluid transfer device 214 is used to transfer some ammonia precursor liquid to start the decomposition in decomposition unit 250, whereupon, step by step, urea boosted solution may be added by fluid transfer device 226.

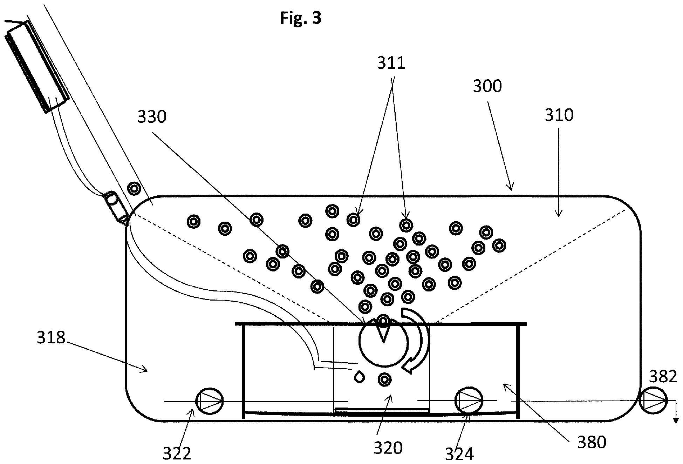

Figure 3 describes another exemplary embodiment of the invention. Here a dissolving and decomposition unit 320 and a buffer tank 380 are integrated into a single cylinder module located in a tank 300. The dissolving and decomposition unit 320 is located in the core of the module and is surrounded by the buffer tank 380. When needed, ammonia precursor granules 311 (optionally with liquid) in a storage compartment 310 can be added to the urea solution in the dissolving and decomposition unit 320 by means of a dosing device 330 to be dissolved and further converted. The urea solution can be transferred from a main chamber 318 of the tank 300 to the dissolving and decomposition unit 320 by a fluid transfer device 322. When the conversion is complete, the resulting effluents are transferred to the buffer tank 380 by a fluid transfer device 324. The ammonia solution with increased concentration in the buffer tank 380 is ready to be sent by a fluid transfer device 382 to e.g. a downstream tank, to the exhaust pipe or to any additional system to store or consume ammonia (not illustrated).

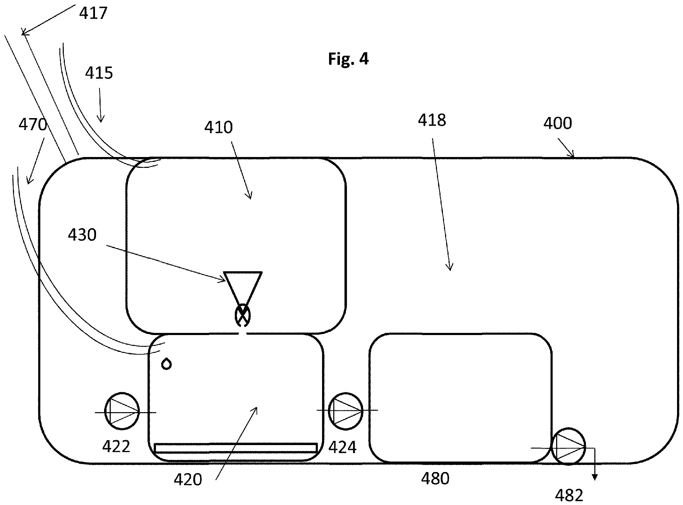

Figure 4 shows another exemplary embodiment. The ammonia precursor granules are stored in a storage compartment 410 in the form of a separated urea storage unit inside a tank 400. When needed, the granules are dispensed into a dissolving and decomposition unit 420 by a dosing device 430. The granules can be prepared as thin particles and may be dissolved at the same temperature as needed for the conversion to ammonia. The granules may be added to the urea storage unit 410 via a filler pipe 415. Enzyme may be added to the dissolving and decomposition unit 420 by a pipe 470. Main chamber 418 is filled with urea solution via filler pipe 417. The urea solution can be transferred from the main chamber 418 of the tank 400 to the dissolving and decomposition unit 420 by a fluid transfer device 422. When the conversion is complete, the resulting effluents are transferred to the buffer tank 480 by a fluid transfer device 424. The ammonia solution with increased concentration in the buffer tank 480 is ready to be sent by a fluid transfer device 482 to e.g. a downstream tank, to the exhaust pipe or to any additional system to store or consume ammonia (not illustrated).

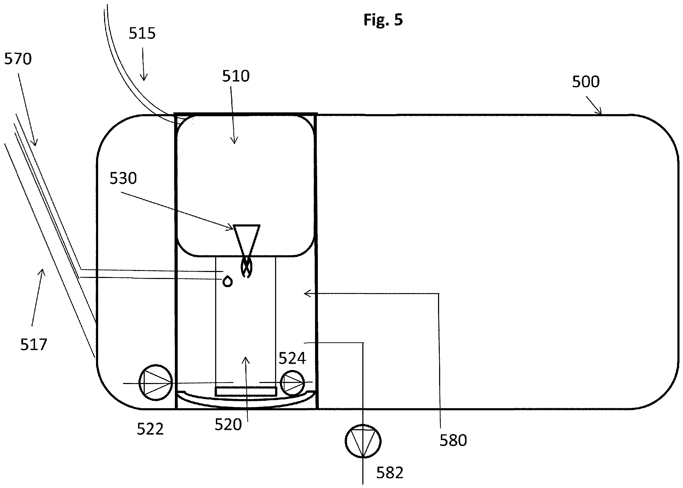

Figure 5 shows a variant of the embodiment of figure 4 where a tank 500 comprises a urea storage unit 510, a dissolving and decomposition unit 520 and a buffer tank 580 which are integrated into a single cylinder module. There is provided a first filler pipe 515 for adding dry ammonia precursor to the urea storage unit 510, and a second filler pipe 517 for filing ammonia precursor liquid to the tank 500. There is provided a dosing device 530 for dosing solid ammonia precursor of the urea storage unit 510 into the decomposition unit 520, and an enzyme transfer pipe 570 for transporting enzyme to the decomposition unit 520. A first fluid transfer device 522 transports ammonia precursor liquid from the tank 500 to the decomposition unit 520, and a second fluid transfer device 524 transfers converted ammonia solution to the buffer tank 580, from where the ammonia solution may be transported by means of a fluid transfer device 582 to any location in the vehicle where the solution is needed.

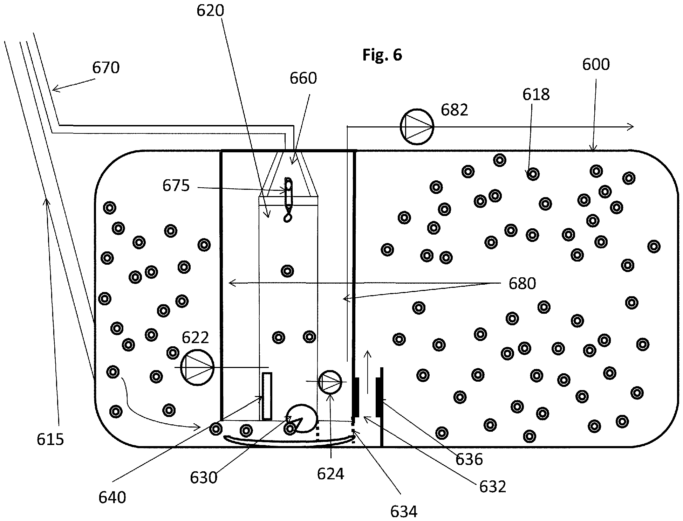

Figure 6 illustrates another ammonia precursor generating system where an enzyme storage unit 660, a decomposition unit 620 and a buffer tank 680 are part of the same module which is arranged in a tank 600. The urea solution is transferred from a main chamber 618 of the tank 600 to the decomposition unit 620 by a fluid transfer device 622. The tank 600 is filled with ammonia precursor liquid and granules via a filler pipe 615. In other words, the main chamber 618 of the tank 600 functions as the storage compartment for storing granules. The enzyme storage unit 660 is filled for example through a filler pipe 670 and contains a bio-agent in suitable form to convert the urea solution into an ammonia solution. For example, as an enzyme, urease can be used to decompose urea, or any other suitable protein sequence. When the conversion is needed, the decomposition unit 620 is filled with the ammonia precursor by the fluid transfer device 622. The ammonia precursor granules 611 can be transferred to the decomposition unit 620 through a channel equipped with a heated transfer tube 632 with a heater 636 in order to generate a flow of liquid and entrain the granules near the dosing device 630 by heat convection or by a thermo-siphon effect. The granules are circulated together with the liquid and are trapped at the end on the tube. The tube is closed at one side by a strainer 634 that allows the liquid to flow, but blocks the passage of the granules. The granules trapped at the end of the tube are then transferred to the decomposition unit 620 by the dosing device 630. Subsequently, a small quantity of enzyme is transferred from the enzyme storage unit 660 to the decomposition unit 620 by an enzyme dosing device 675. Optionally the enzyme storage unit 660 may be provided with thermal conditioning element. When the conversion is complete, the resulting effluents are transferred to the buffer tank 680 by a fluid transfer device 624.

The solution in the buffer tank 680 is ready to be sent by a fluid transfer device 682 to a downstream tank, to the exhaust pipe or to any additional system to store or consume ammonia (not illustrated). The ammonia buffer tank 680 can also communicate with an ammonia-hydrogen conversion unit that subsequently communicates with a hydrogen fuel cell where the hydrogen is then converted into a power source such as electricity (not illustrated), as in the embodiment of figure 1. Alternatively, the ammonia solution can be used in a direct ammonia fuel cell (not illustrated).

According to a variant of the embodiments of figure 1-6, fluid transfer device 122, 322, 422, 522, 622 and the dosing device 130, 330, 430, 530, 630 can be combined in a single component to feed continuously liquid ammonia precursor and granules in a given proportion, simplifying the setup.

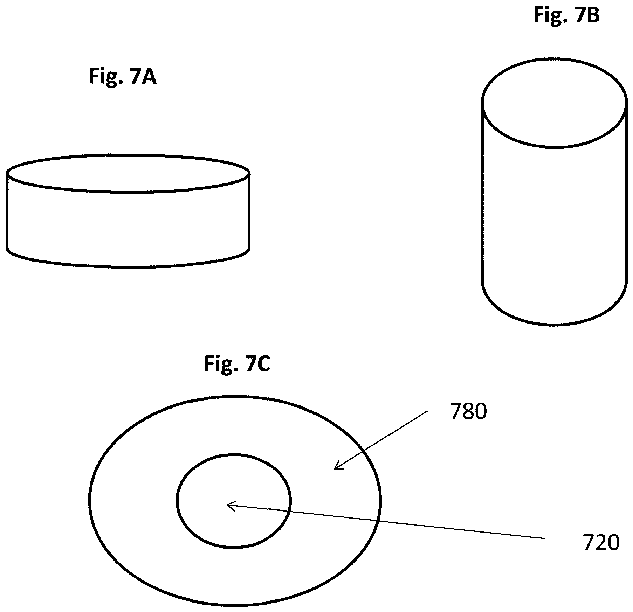

Figures 7A and 7B illustrate two variants of a suitable module in which the dissolving and decomposition compartment and buffer compartment may be arranged. The module can be designed as a multi-compartment container with different shapes, such as a barrel shape illustrated in figure 7A or a tower shape illustrated in figure 7B. For a typical barrel shape the height will be less than the diameter. Both shapes may be composed of a central cylindrical compartment forming the decomposition unit 720 and a surrounding compartment forming the buffer tank 780, as illustrated in figure 7C. This has the advantage that the warm decomposition unit 720 is separated from the cold main chamber by the buffer compartment 780. In addition, with the preferred setup, the effluents in buffer tank 780 will be at an intermediate temperature between the temperature of the cold chamber of the main tank and the temperature of the decomposition unit 720, and will also contribute to limit the heat losses from the decomposition unit 720. Also, in case of leakage of the buffer solution containing ammonia, this irritating substance will stay in the main tank containing urea. The more diluted ammonia solution is then less dangerous to the environment. An enzyme storage unit and/or a urea storage unit may be located on the top of the module and may be integrated in the module.

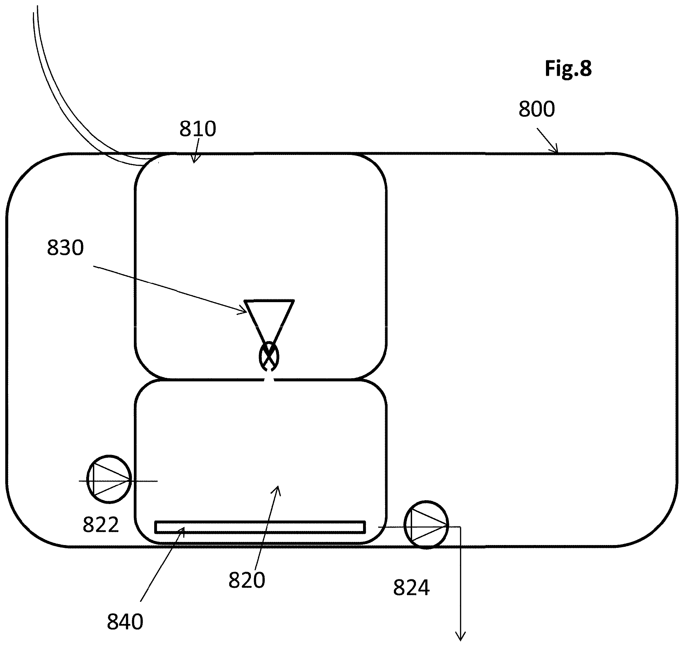

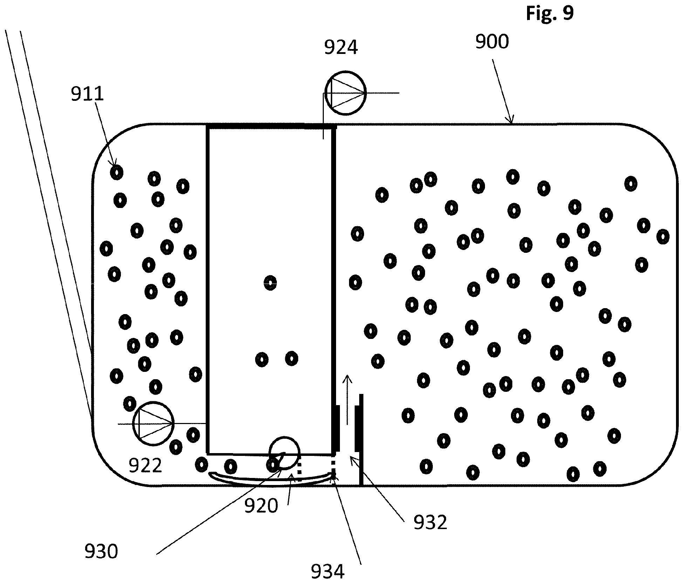

Figure 8 illustrates a further embodiment where ammonia precursor granules stored into a urea storage unit 810, are transferred into a warm dissolving compartment 820 by a dosing device 830 where the granules can be thermally dissolved in a urea solution. The urea storage unit 810 and the dissolving compartment 820 are arranged in a tank 800 which can be filled with an aqueous urea solution which is transferred by a liquid transfer device 822 to the dissolving compartment 820. The boosted urea solution in the dissolving compartment 820 can be transferred by a further liquid transfer device 824 to a location in a vehicle where the boosted urea solution is needed. Alternatively, as illustrated in figure 9, ammonia precursor granules can be transferred to a warm chamber 920 through a channel equipped with a heated transfer tube 932 in order to generate liquid circulation by convection or by a thermo-siphon method. The granules are circulated together with the liquid and trapped at the end on the tube by a strainer 934. The granules 911 are transferred to the warm chamber 920 by a dosing device 930. The boosted (or more concentrated) urea solution in the warm chamber is ready to be sent by a fluid transfer device 924 to the exhaust pipe and SCR catalyst or to any additional system to store or consume urea (not illustrated). Optionally, the dosing device 930 and the fluid transfer device 922 may be combined in a single component arranged to send granules and liquid ammonia precursor according to a set proportion to the warm chamber 920, simplifying the setup.

In the exemplary embodiments illustrated above, when using AdBlue® as the ammonia precursor liquid, the total urea concentration in the dissolving compartment may be progressively increased to 55% by weight after dissolution of the granules in AdBlue®. In other words, 1 kg of solution contains 450 g of water and 550 g of urea, from which 217 g (32.5% of (450 g + 217 g)) originate from the eutectic AdBlue® and 333 g have been added in the form of solid ammonia precursor granules per kg of solution. While a conventional SCR system would require 45 litre useful volume to reach a driving range of 30000 km (corresponding for instance to the maintenance interval) with a consumption of 0.15 litre of AdBlue® per 100 km, embodiments of a system boosted with granules would require only about 27 litre of useful volume for the same driving range. Accordingly, the weight of the full system would be reduced by about 20 kg.

In another exemplary embodiment, the total urea concentration may be progressively increased to 76.9% by weight after dissolution of the granules in AdBlue®. So 1 kg of solution contains 231 g of water and 769 g of urea, from which 111 g (32.5% of (231 g + 111 g)) originate from the eutectic AdBlue® and 658 g have been added in the form of solid ammonia precursor granules per kg of solution. While a conventional SCR system would require 45 litre useful volume to reach a driving range of 30000 km (corresponding for instance to the maintenance interval) with a consumption of 0.15 litre of AdBlue® per 100 km, embodiments of the system boosted with granules would only require about 20 litre of useful volume for the same driving range. Accordingly, the weight of the full system would be reduced by about 25 kg.

In the example of a fuel cell feeding system, a vehicle equipped with a prior art system of 70 litre useful volume based on AdBlue® and consuming 28 litre/100 km has a driving range of 250 km, while a system of the same useful volume in accordance with embodiments of the invention and filled with the boosted solution having an urea concentration boosted at 55% in weight as set out above, will reach a driving range of about 420 km, and a system filled with the boosted solution having an urea concentration boosted at 76,9% in weight as set out above, will reach a driving range close to 600 km.

In an exemplary embodiment a granule contains solid ammonia precursor, and has a coating adapted to be dissolved in an ammonia precursor liquid. The solid ammonia precursor is preferably solid urea, but ammonia precursor granules may contain other materials than urea, such as ammonium carbamate which is also a solid that can dissolve in water and generate ammonia, and more generally ammonia salts. Preferably the coating is adapted to be thermally dissolved in an ammonia precursor liquid. The coating of the ammonia precursor granules may be made of any one or more of the following materials: polyvinylidene chloride (PVDC), linear low density polyethylene (LLDPE), certain grades of ethylene vinyl alcohol (EVOH), certain grades of polyvinyl alcohol (PVOH), biaxially oriented polypropylene (BOPP), cyclic olefin polymer (COC), polyethylene naphthalate (PEN), liquid-crystal polymers (LCPs, a class of aromatic polyester polymers), polypropylene (PP), and polyethylene terephthalate blends (PET/PE, PET/PVDC/PE, PET/PVOH/PE, PET/EVOH/PE). Whilst the principles of the invention have been set out above in connection with specific embodiments, it is to be understood that this description is merely made by way of example and not as a limitation of the scope of protection which is determined by the appended claims.

## Claims

1. A tank (100) for storing a first ammonia precursor solution having a first ammonia precursor concentration, said tank comprising an ammonia precursor generating system for generating a second ammonia precursor solution having a second ammonia precursor concentration, the second ammonia precursor concentration being higher than the first ammonia precursor concentration, said ammonia precursor generating system comprising the following compartments and means that are located in the tank (100):
a storage compartment (110) storing at least ammonia precursor granules;
a dissolving compartment (120);
a transfer means (130) configured for transferring ammonia precursor granules from said storage compartment (110) to said dissolving compartment (120);
a fluid transfer device (122) configured for transferring first ammonia precursor solution to said dissolving compartment (120);
said dissolving compartment (120) being configured for generating second ammonia precursor solution by dissolving ammonia precursor granules in first ammonia precursor solution.

2. The tank of claim 1, wherein said transfer means (130) is a dosing device (130) configured for dosing a number of ammonia precursor granules to be inserted in the dissolving compartment (120).

3. The tank of claim 1 or 2, wherein said dissolving compartment (120) comprises a trigger device arranged for triggering the dissolving the ammonia precursor granules in the ammonia precursor liquid.

4. The tank of claim 3, wherein said trigger device is a heater arranged for heating the ammonia precursor solution in the dissolving compartment (120) and configured for dissolving the ammonia precursor granules in the ammonia precursor liquid.

5. The tank of any one of the previous claims, wherein the storage compartment (110) stores ammonia precursor granules containing solid urea.

6. The tank of any one of the previous claims, wherein the storage compartment (110) stores ammonia precursor granules having a coating, said coating being adapted to be thermally dissolved in the ammonia precursor liquid.

7. The tank of claim 6, wherein the storage compartment (110) stores the ammonia precursor granules in a liquid, and said coating is such that the coating is not dissolved in the liquid in the storage compartment.

8. The tank of any one of the previous claims, further comprising a filler pipe in connection with the storage compartment (110) for filling the storage compartment with ammonia precursor granules; wherein said filler pipe is optionally also intended for filling said storage compartment (110) with a ammonia precursor liquid, and/or wherein the storage compartment (110) is arranged for allowing said ammonia precursor liquid to flow to the dissolving compartment (120).

9. The tank of any one of the previous claims, wherein the dissolving compartment (120) is provided with a decomposition activator device configured to convert ammonia precursor solution to ammonia solution in the dissolving compartment (120).

10. The tank of claim 9, wherein the decomposition activator device comprises an enzyme storage unit (160) configured to store an enzyme, and an enzyme transfer means configured for transferring enzyme to the dissolving compartment (120), said enzyme being adapted to convert ammonia precursor to ammonia.

11. The tank of any one of the previous claims, comprising a decomposition compartment (120) provided with a decomposition activator device configured to convert ammonia precursor solution to ammonia solution, and a transfer means configured for transferring ammonia precursor solution from the dissolving compartment to the decomposition compartment; wherein the decomposition activator device optionally comprises an enzyme storage unit (160) configured to store an enzyme, an enzyme transfer means (170, 175) configured for transferring enzyme to the decomposition compartment (120), said enzyme being adapted to convert ammonia precursor to ammonia, and a heater (140).

12. The tank of any one of the claims 9-11, further comprising a buffer compartment (180) for storing the ammonia solution; wherein the buffer compartment (180) optionally surrounds the dissolving compartment (120), and/or wherein the buffer compartment (180) and the dissolving compartment (120) are integrated in a single module.

13. The tank of any one of the previous claims, further comprising a conversion unit (190) for converting ammonia into hydrogen; and /or a controller configured for controlling the transfer means such that granules are transferred to the dissolving unit upon request, as needed.

14. SCR system comprising a tank (100) according to any one of the previous claims.

15. Fuel cell system comprising a tank (100) according to any one of claims 1 to 13.

## Patentansprüche

1. Tank (100) zum Lagern einer ersten Ammoniakvorläuferlösung mit einer ersten Ammoniakvorläuferkonzentration,
wobei der Tank ferner ein Ammoniakvorläufererzeugungssystem zum Erzeugen einer zweiten Ammoniakvorstufenlösung mit einer zweiten Ammoniakvorläuferkonzentration umfasst,
wobei die zweite Ammoniakvorläuferkonzentration höher ist als die erste Ammoniakvorläuferkonzentration, wobei das Ammoniakvorläufererzeugungssystem ferner die folgenden Abteile und Mittel umfasst, die in dem Tank (100) angeordnet sind:
ein Speicherabteil (110), das mindestens Ammoniakvorläufergranulat speichert;
ein Auflösungsabteil (120);
eine Übertragungseinrichtung (130), die zum Übertragen von Ammoniakvorläufergranulat aus dem Speicherabteil (110) zu dem Auflösungsabteil (120) konfiguriert ist;
eine Fluidüberführungsvorrichtung (122), die zum Überführen einer ersten Ammoniakvorläuferlösung in das Auflösungsabteil (120) konfiguriert ist; wobei das Auflösungsabteil (120) zum Erzeugen einer zweiten Ammoniakvorläuferlösung durch Auflösen von Ammoniakvorläufergranulat in der ersten Ammoniakvorläuferlösung konfiguriert ist.

2. Tank nach Anspruch 1, wobei die Transfereinrichtung (130) eine Dosiervorrichtung (130) ist, die zum Dosieren einer Anzahl von Ammoniakvorläufergranulat konfiguriert ist, die in das Löseabteil (120) eingeführt werden sollen.

3. Tank nach Anspruch 1 oder 2, wobei das Auflösungsabteil (120) eine Auslösevorrichtung umfasst, die zum Auslösen des Lösens der Ammoniakvorläufergranulate in der Ammoniakvorläuferflüssigkeit eingerichtet ist.

4. Tank nach Anspruch 3, wobei die Auslösevorrichtung eine Heizvorrichtung ist, die zum Erwärmen der Ammoniakvorläuferlösung in dem Löseabteil (120) angeordnet ist und ferner zum Auflösen des Ammoniakvorläufergranulats in der Ammoniakvorläuferflüssigkeit eingerichtet ist.

5. Tank nach einem der vorhergehenden Ansprüche, wobei das Speicherabteil (110) Ammoniakvorläufergranulate enthält, die festen Harnstoff enthalten.

6. Tank nach einem der vorhergehenden Ansprüche, wobei das Speicherabteil (110) Ammoniakvorläufergranulat mit einer Beschichtung speichert, wobei die Beschichtung dafür ausgelegt ist, in der Ammoniakvorläuferflüssigkeit thermisch gelöst zu werden.

7. Tank nach Anspruch 6, wobei das Speicherabteil (110) ferner die Ammoniakvorläufergranulate in einer Flüssigkeit speichert und die Beschichtung derart ausgelegt ist, dass die Beschichtung nicht in der Flüssigkeit in dem Speicherabteil gelöst ist.

8. Tank nach einem der vorhergehenden Ansprüche, ferner umfassend ein Füllrohr in Verbindung mit dem Speicherabteil (110) zum Füllen des Speicherabteils mit Ammoniakvorläufergranulat; wobei das Einfüllrohr optional auch dazu bestimmt ist, das Speicherabteil (110) mit einer Ammoniakvorläuferflüssigkeit zu füllen, und / oder wobei das Speicherabteil (110) dazu angeordnet ist, um zu ermöglichen, dass die Ammoniakvorläuferflüssigkeit zu dem Auflösungsabteil (120) fließt.

9. Tank nach einem der vorhergehenden Ansprüche, wobei das Auflösungsabteil (120) mit einer Zersetzungsaktivatorvorrichtung versehen ist, die konfiguriert ist, um die Ammoniakvorstufenlösung in Ammoniaklösung in dem Auflösungsabteil (120) umzuwandeln.

10. Tank nach Anspruch 9, wobei die Zersetzungsaktivatorvorrichtung als eine Enzymspeichereinheit (160) ausgebildet ist, die zum Speichern eines Enzyms konfiguriert ist, und eine Enzymtransfereinrichtung zum Übertragen von Enzym zu dem Löseabteil (120) umfasst, wobei das Enzym zum Umwandeln der Ammoniakvorstufe zu Ammoniak angepasst ist.

11. Tank nach einem der vorhergehenden Ansprüche, ferner umfassend ein Zersetzungskompartiment (120), das mit einer Zersetzungsaktivatorvorrichtung versehen ist, die konfiguriert ist, die Ammoniakvorläuferlösung in eine Ammoniaklösung umzuwandeln, und eine Transfereinrichtung, die zum Überführen von Ammoniakvorläuferlösung von dem Zersetzungskompartiment zu dem Auflösungsabteil (120) ausgebildet ist;
wobei das Auflösungsabteil eine Aktivatorvorrichtung umfasst, ferner optional eine Enzymspeichereinheit (160), die konfiguriert ist, um ein Enzym zu speichern, ein Enzymtransfermittel (170, 175), das zum Übertragen von Enzym zu dem Zersetzungsabteil (120) konfiguriert ist, um den Ammoniakvorläufer in Ammoniak umzuwandeln, und ferner umfassend eine Heizung (140).

12. Tank nach einem der Ansprüche 9 bis 11, wobei der Tank ferner ein Pufferabteil (180) zum Speichern der Ammoniaklösung umfasst; wobei das Pufferabteil (180) optional das Auflösungsabteil (120) umgibt und / oder wobei das Pufferabteil (180) und das Auflösungsabteil (120) in einem einzelnen Modul integriert sind.

13. Tank nach einem der vorhergehenden Ansprüche, wobei der Tank ferner eine Umwandlungseinheit (190) zum Umwandeln von Ammoniak in Wasserstoff umfasst; und / oder ferner eine Steuerung, die konfiguriert ist, um die Übertragungseinrichtung so zu steuern, dass das Granulat nach Bedarf bei Bedarf zu der Auflösungseinheit übertragen wird.

14. SCR-System mit einem Tank (100) nach einem der vorhergehenden Ansprüche.

15. Brennstoffzellensystem mit einem Tank (100) nach einem der Ansprüche 1 bis 13.

## Revendications

1. Un réservoir (100) pour stocker une première solution de précurseur d'ammoniac ayant une première concentration en précurseur d'ammoniac, ledit réservoir comprenant un système de génération de précurseur d'ammoniac pour générer une deuxième solution de précurseur d'ammoniac ayant une deuxième concentration en précurseur d'ammoniac, la concentration en deuxième précurseur d'ammoniac étant supérieure à la première concentration en précurseur d'ammoniac, ledit système de génération de précurseur d'ammoniac comprenant les compartiments et moyens suivants qui sont situés dans le réservoir (100) :
un compartiment de stockage (110) stockant au moins des granulés de précurseur d'ammoniac ;
un compartiment de dissolution (120) ;
un moyen de transfert (130) configuré pour transférer des granulés de précurseur d'ammoniac depuis ledit compartiment de stockage (110) vers ledit compartiment de dissolution (120) ;
un dispositif de transfert de fluide (122) configuré pour transférer la première solution de précurseur d'ammoniac vers ledit compartiment de dissolution (120) ;
ledit compartiment de dissolution (120) étant configuré pour générer une deuxième solution de précurseur d'ammoniac en dissolvant des granulés de précurseur d'ammoniac dans la première solution de précurseur d'ammoniac.

2. Le réservoir selon la revendication 1, dans lequel ledit moyen de transfert (130) est un dispositif de dosage (130) configuré pour doser un certain nombre de granulés de précurseur d'ammoniac destinés à être insérés dans le compartiment de dissolution (120).

3. Le réservoir selon la revendication 1 ou la revendication 2, dans lequel ledit compartiment de dissolution (120) comprend un dispositif de déclenchement agencé pour déclencher la dissolution des granulés de précurseur d'ammoniac dans le liquide précurseur d'ammoniac.

4. Le réservoir selon la revendication 3, dans lequel ledit dispositif de déclenchement est un dispositif de chauffage agencé pour chauffer la solution de précurseur d'ammoniac dans le compartiment de dissolution (120) et configuré pour dissoudre les granulés de précurseur d'ammoniac dans le liquide précurseur d'ammoniac.

5. Le réservoir selon l'une quelconque des revendications précédentes, dans lequel le compartiment de stockage (110) stocke des granulés de précurseur d'ammoniac contenant de l'urée solide.

6. Le réservoir selon l'une quelconque des revendications précédentes, dans lequel le compartiment de stockage (110) stocke des granulés de précurseur d'ammoniac ayant un revêtement, ledit revêtement étant adapté pour être dissous thermiquement dans le liquide précurseur d'ammoniac.

7. Le réservoir selon la revendication 6, dans lequel le compartiment de stockage (110) stocke les granulés de précurseur d'ammoniac dans un liquide, et ledit revêtement est tel que le revêtement n'est pas dissous dans le liquide dans le compartiment de stockage.

8. Le réservoir selon l'une quelconque des revendications précédentes, comprenant en outre un tuyau de remplissage en liaison avec le compartiment de stockage (110) pour remplir le compartiment de stockage avec des granulés de précurseur d'ammoniac ; ledit tuyau de remplissage étant également, optionnellement, prévu pour remplir ledit compartiment de stockage (110) avec un liquide précurseur d'ammoniac, et / ou le compartiment de stockage (110) étant agencé pour permettre au liquide précurseur d'ammoniac de s'écouler dans le compartiment de dissolution (120).

9. Le réservoir selon l'une quelconque des revendications précédentes, dans lequel le compartiment de dissolution (120) est muni d'un dispositif activateur de décomposition configuré pour convertir une solution de précurseur d'ammoniac en une solution d'ammoniac dans le compartiment de dissolution (120).

10. Le réservoir selon la revendication 9, dans lequel le dispositif activateur de décomposition comprend une unité de stockage enzymatique (160) configurée pour stocker une enzyme, et un moyen de transfert d'enzyme configuré pour transférer l'enzyme au compartiment de dissolution (120), ladite enzyme étant adaptée pour convertir un précurseur d'ammoniac en ammoniac.

11. Le réservoir selon l'une quelconque des revendications précédentes, comprenant un compartiment de décomposition (120) pourvu d'un dispositif activateur de décomposition configuré pour convertir une solution de précurseur d'ammoniac en solution d'ammoniac, et un moyen de transfert configuré pour transférer la solution de précurseur d'ammoniac du compartiment de dissolution au compartiment de décomposition ; le dispositif activateur de décomposition comprenant facultativement une unité (160) de stockage d'enzyme configurée pour stocker une enzyme, un moyen de transfert d'enzyme (170, 175) configuré pour transférer l'enzyme dans le compartiment de décomposition (120), ladite enzyme étant adaptée pour convertir le précurseur d'ammoniac en ammoniac, et un dispositif de chauffage (140).

12. Le réservoir selon l'une quelconque des revendications 9 à 11, comprenant en outre un compartiment tampon (180) pour stocker la solution d'ammoniac ; le compartiment tampon (180), optionnellement, entourant le compartiment de dissolution (120), et / ou le compartiment tampon (180) et le compartiment de dissolution (120) étant intégrés dans un seul module.

13. Le réservoir selon l'une quelconque des revendications précédentes, comprenant en outre une unité de conversion (190) pour convertir l'ammoniac en hydrogène ; et / ou un contrôleur configuré pour commander les moyens de transfert de telle sorte que les granulés sont transférés à l'unité de dissolution sur demande, selon les besoins.

14. Système SCR comprenant un réservoir (100) selon l'une quelconque des revendications précédentes.

15. Système de pile à combustible comprenant un réservoir (100) selon l'une quelconque des revendications 1 à 13.
